# EUROPEAN PATENT SPECIFICATION

(11) **EP 0 686 141 B1**
(45) Date of publication and mention of the grant of the patent: **09.12.1998**
(21) Application number: 94908531.0
(22) Date of filing: 25.02.1994
(51) Int. Cl.: C07C 201/08, C07C 205/06, C07C 205/12, C07C 205/44, C07C 205/22, C07C 205/23, C07C 205/37, C07C 205/55

(54) **NEW PROCESS FOR THE NITRATION OF AROMATIC COMPOUNDS IN MILD, NON-CORROSIVE CONDITIONS**
VERFAHREN ZUR NITRIERUNG VON AROMATISCHEN VERBINDUNGEN VERBINDUNGEN UNTER MILDEN, NICHT KORROSIVEN BEDINGUNGEN
NOUVEAU PROCEDE DE NITRATATION DE COMPOSES AROMATIQUES DANS DES CONDITIONS DOUCES ET NON CORROSIVES

(30) Priority: 26.02.1993 PT 10120793
(43) Date of publication of application: 13.12.1995
(73) Proprietor: INSTITUTO NACIONAL DE ENGENHARIA E TECNOLOGIA INDUSTRIAL, 1699 Lisboa Codex (PT)
(72) Inventor: SILVA GIGANTE CARVALHEIRO, Bárbara, Manuela, P-1600 Lisboa (PT); LASZLO, Pierre, F-78470 Saint-Rémy-les-Chevreuse (FR); CORN LIS, André, B-4100 Seraing (BE); VIDAL DE OLIVEIRA BAPTISTA MARCELO CURTO, Maria J., P-2780 Oeiras (PT)
(74) Representative: Arantes e Oliveira, Joao de
(86) International application number: PT9400001
(87) International publication number: WO9419310

(56) References cited:
- EP-A- 0 356 091
- US-A- 5 210 339
- CHEMICAL ABSTRACTS, vol. 111, no. 1, 3 July 1989, Columbus, Ohio, US; abstract no. 006976, CORNELIS A ET AL 'Regioselective liquid-phase toluene nitration with modified clays as catalysts' & CHEM. LETT. (CMLTAG,03667022);88; (11); PP.1839-42 UNIV. LIEGE;LAB. CHIM. FINE INTERFACES; LIEGE; BELG. (BE)
- CHEMICAL ABSTRACTS, vol. 107, no. 17, 26 October 1987, Columbus, Ohio, US; abstract no. 153994, LASZLO P ET AL 'Vastly improved para preference in the nitration of halobenzenes' cited in the application & J. ORG. CHEM. (JOCEAH,00223263);87; VOL.52 (12); PP.2407-10 UNIV. LIEGE;INST. CHIM. ORG.; LIEGE; 4000; BELG. (BE)
- CHEMICAL ABSTRACTS, vol. 111, no. 7, 14 August 1989, Columbus, Ohio, US; abstract no. 057138, CORNELIS A ET AL 'A procedure for quantitative regioselective nitration of aromatic hydrocarbons in the laboratory' & TETRAHEDRON LETT. (TELEAY,00404039);88; VOL.29 (46); PP.5909-12 UNIV. LIEGE;INST. CHIM. ORG. B6; LIEGE; 4000; BELG. (BE)
- CHEMICAL ABSTRACTS, vol. 111, no. 5, 31 July 1989, Columbus, Ohio, US; abstract no. 038945, LASZLO P ET AL 'Regioselective nitration of aromatic hydrocarbons by metallic nitrates on the K10 montmorillonite under Menke conditions' cited in the application & CHEM. LETT. (CMLTAG,03667022);88; (11); PP.1843-6 UNIV. LIEGE;INST. CHIM. ORG.; LIEGE; 4000; BELG. (BE)
- CHEMICAL ABSTRACTS, vol. 083, no. 3, 21 July 1975, Columbus, Ohio, US; abstract no. 027761, FUKUNAGA K 'Nitration and utilization of nitro compounds. II. Reaction of acyl nitrates formed from metal nitrates or nitric acid in carboxylic anhydrides with some aromatic hydrocarbons' & NIPPON KAGAKU KAISHI (NKAKB8);74; (11); PP.2231-4 YAMAGUCHI UNIV.;FAC. ENG.; UBE; JAPAN

## Description

This invention describes a new process for nitration of aromatic compounds using silicates, optionally modified with metal nitrates, and reduced amounts of nitric acid under mild, non-corrosive conditions.

### BACKGROUND OF THE INVENTION

By far the most common industrial process for nitration of aromatic compounds is the sulfuric acid catalyzed reaction with nitric acid, also called the mixed acid nitration, which is frequently conducted at high temperatures. More particularly, nitration of deactivated aromatic compounds is carried out in nitric acid-oleum. See, G. Ohla, *et al., Nitration: Methods and Mechanisms,* VCH, New York, 1989. The use of aggressive sulfo-nitric mixtures and the temperature requirements make such nitration processes costly in terms of capital investment and energy. See, Audley, *et al., J. Chem. Soc., Chem. Commun.,* 1053-1055 (1982) and Seifert, *J. Org. Chem.,* **28**:125-129 (1963). Additionally, the use of aggressive sulfo-nitric mixtures and elevated temperatures is particularly hazardous.

In view of the above disadvantages and safety considerations, considerable effort has focused on discovering means of carrying out nitration processes under mild, non-corrosive conditions.

The nitration of aromatics with acyl nitrates formed from metal nitrates or nitric acid in carboxylic anhydrides was described by Fukunaga, *Nippon Kagaku Kaishi,* (11), 2231-2234 (1974) [*CA,* Vol. 083, 21 July 1975, abstract no. 027761], to produce mononitrated products, but has the disadvantage of forming acyloxylated by-products. The use of acyl nitrates in the presence of a zeolite or a clay has been described for the para-selective mononitration of mono-substituted [alkyl or aryl] aromatics (see EP-A-0 356 091, 1989, priority 1988), but has the disadvantages of requiring the preparation and manipulation of explosive acyl nitrates and being limited to mononitration of activated aromatics.

A procedure for the quantitative nitration of aromatic hydrocarbons using a clay modified with cupric nitrate has been reported. However, this process only gives rise to products of mononitration. No polynitration products are observed. See, Cornélis, *et al., Tetrahedron Lett.,* **29**:5657-5660 and 5909-5912 (1988); Laszlo, *et al., Chem. Lett.,* 1843-1846 (1988) and Laszlo, *et al., J. Org. Chem.,* **52:**2407-2410 (1987).

While the use by others of metal nitrate impregnated clays can be an advantage when only mononitrated products are desired, the method cannot be applied for the production of polynitrated products such as 1,3-dinitrobenzene, dinitrotoluenes, dinitronaphthalenes, dinitro-p-cresol, 12,14-dinitrodehydroabietic acid or nitro musks such as musk xylol, *i.e.* 5-t-butyl-1,3-dimethyl--2,4,6-trinitrobenzene. Moreover, application of the above mentioned process to the nitration of unactivated aromatic compounds, such as benzene or other aromatic compounds possessing deactivating substituents, leads to low yields of the mononitration products.

The above polynitroaromatic compounds are important intermediates for the production of chemicals with many industrial applications and are used in the preparation of polymers, explosives, dyes, pharmaceuticals, agrochemicals and fragrances. Aromatic diisocyanates, which are commonly used in the production of polymers, are currently produced from the corresponding dinitro compounds which are in turn produced using the aggressive sulfo-nitric mixture. Nitrobenzene is produced by nitration of benzene with mixed acid at 50° to 160°C. 1,3-Dinitrobenzene is produced in 82% yield by subsequent nitration of nitrobenzene with mixed acid at 60-90°C. See, *Ullmann's Encyclopedia of Industrial Chemistry,* 5^{th} Ed., VCH, NY, 1987, Vol. A17, p. 415-418. 2,4-Dinitrotoluene and 2,6-dinitrotoluene are produced directly from toluene with mixed acid as a 80:20 mixture. See, Josef Meissner GmbH & Co., DE 2 921 487 (1979). Both this mixture and the 2,4-dinitrotoluene isolated thereof are intermediates for the manufacture of toluenediisocyanate (TDI), an important raw material for the manufacture of polyurethanes and explosives. See, *Ullmann's Encyclopedia of Industrial Chemistry,* 5^{th} Ed., VCH, NY, 1987, Vol. A17, p. 422; Chadwick, *et al.,* in *Kirk-Othmer* *Encyclopedia of Chemical Technology,* 3^{rd} Ed., Wiley Interscience, NY, 1983, Vol. 23, p. 265. 1,5-Dinitronaphthalene, an intermediate in the manufacture of diisocyanates, and 1,8-dinitronaphthalene, which is used in the production of dyes, are obtained by nitration of naphthalene with mixed acid. A mixture of isomers is obtained in which the 1,8-isomer predominates. See, *Ullmann's Encyclopedia of Industrial Chemistry,* 5^{th} Ed., VCH, NY, 1987, Vol. A17, p. 425. 12,14-Dinitrodehydroabietic acid, an intermediate in the syntheses of physiologically active derivatives possessing the hydrophenanthrene skeleton, is also of industrial interest, and is also produced by nitration with mixed acid. See, Fieser, *et al., J. Am. Chem. Soc.,* **60:**159-170 (1951). Similarly, the nitro musks, such as musk tibetene and musk xylol, which are important commodities in the perfumery industry, are currently produced using the aggressive sulfo-nitric mixture. See, Fuson, *et al.,* J. *Org. Chem.,* **13:**587-595 (1947) and Carpenter, *et al., J. Org. Chem.,* **16**:586-617 (1951).

Nitro derivatives of aromatic heterocycles also find application in several fields such as pharmaceutical chemistry, corrosion inhibition, dye production and agrochemistry. 2-Nitroimidazole(azomycin) possesses antimicrobial activity and is an important intermediate in the preparation of 2-nitroimidazolyl carbamates, potential protozoacides; however, its synthesis is difficult and requires reaction of 2-aminoimidazole sulfate with sodium nitrite in the presence of excess copper sulfate. See Beaman, *et al., J. Am. Chem. Soc.,* **87:** 389-390 (1965). 4-Nitroimidazole is more readily obtained by direct nitration of imidazole with nitric acid. See H. Spaenig *et al.,* BASF, DE 2 208 924 (1972). Furfural is nitrated with nitric acid in acetic anhydride solution at 25-40°C to give 5-nitro-2-furaldehyde diacetate and then used directly for the production of derived drugs. See Sanders *et al., Ind. Eng. Chem.,* **47**:358-367 (1955) and *Ullmann's Encyclopedia of Industrial Chemistry,* 5^{th} Ed., VCH, NY, 1987, Vol. A17, p. 452.

Thiophene and derivatives are nitrated via mixed acid or via acetyl nitrate processes. *See Ullmann's Encyclopedia of Industrial Chemistry,* 5^{th} Ed., VCH, NY, 1987, Vol. A17, p. 452-453.

A method for the polynitration of arenes at low temperature has been reported recently. See, Suzuki, *et al., J. Chem. Soc., Chem. Commun.,* 1049-1050 (1991). This method involves nitration with nitrogen dioxide, in the presence of ozone and a catalyst, such as methane sulfonic acid. Application of this method to the synthesis of musk xylol proceeds in 80% yield. However, this process has the disadvantage of using nitrogen dioxide, a highly toxic gas.

What is needed in the art is a process to selectively prepare mono- or polynitrated aromatic compounds which provides high yields, is economical in terms of energy requirements and capital investment, is efficient in reducing the number of synthetic steps to prepare a particular compound, mild to the substrate as a whole, minimizes the formation of oxidation products, and safe.

Quite surprisingly, the present invention fulfills these and other related needs.

### SUMMARY OF THE INVENTION

The present invention provides new processes for the nitration of aromatic substrates. In one process, an aromatic substrate is reacted with a silicate, optionally modified with a metal nitrate, in the presence of an acid anhydride and an organic solvent, followed by the addition of reduced amounts of nitric acid. Isolation of the nitrated products involves filtration, washing and solvent removal. This process can produce either mono- or polynitrated aromatic compounds depending upon the conditions selected and the nature of the aromatic compound. The present invention also provides related processes for the nitration of strongly activated aromatic substrates using only silicates modified with metal nitrate in the presence of acid anhydrides and an organic solvent.

All of these processes provide advantages in yield, cost, and safety over the industrial methods of nitration using mixed acids.

### DETAILED DESCRIPTION

### Definitions

The terms "aromatic compound" and "aromatic substrate" refer to a group of unsaturated cyclic hydrocarbons containing one or more rings. The rings are typified by benzene which has a 6-carbon ring containing three double bonds. Compounds containing multiple rings may have the rings fused together or linked covalently. Examples of such multiple ring aromatic compounds are naphthalene, biphenyl and anthracene. The terms "aromatic compound" and "aromatic substrate" also refer to those compounds described above which contain heteroatoms, for example, pyridine, quinoline and quinoxaline. Other aromatic compounds include certain 5-membered cyclic compounds such as furan and thiophen, including those containing multiple rings such as benzofuran, benzothiophen and indole. As used herein, the terms "aromatic compound" and "aromatic substrate" also refer to polycyclic compounds wherein at least one ring is an aromatic ring as described above. Additionally, any of the aromatic rings described herein may be optionally substituted with halogen atoms, or other groups such as amine (-NH₂), alkyl (-R), hydroxy (-OH), carboxaldehyde (-CHO), alkoxy (-OR).

The term "activated", when used to describe an aromatic compound or substrate, refers to compounds having at least one electron donating substituent such as alkyl (-R) or aryl (-Ar). Examples of activated aromatic substrates and compounds are toluene, xylene, biphenyl, or naphthalene. Within the group of activated aromatic compounds or substrates are those which are "strongly activated". The "strongly activated" aromatic compounds or substrates are those having at least one substituent containing an unshared pair of electrons on the atom connected to the aromatic ring. Such groups include -OH, -OR, -NH₂, -NHR, -NR₂, -NHCOR, -OCOR or the like. Examples of strongly activated aromatic substrates and compounds are phenol, anisole, cresol or aniline.

The term "deactivated", when used to describe an aromatic substrate or compound, refers to compounds having electron withdrawing substituents such as carboxaldehyde (-CHO), nitro (-NO₂), carboxylic acid (-COOH), sulfonic acid (-SO₂H), halogens (-F, -Cl, -Br and -I), cyano'(-CN), or trifluoromethyl (-CF₃). Examples of deactivated aromatic substrates or compounds are benzonitrile, benzoic acid, benzaldehyde, chloronaphthalene or bromobenzene.

The term "unactivated", when used to describe an aromatic substrate or compound, refers to compounds which are unsubstituted. Examples of such compounds are benzene, naphthalene, phenanthrene or anthracene.

As used herein, the term "acid anhydride" refers to anhydrides of lower carboxylic acids and sulphonic acids such as acetic acid, propionic acid, butanoic acid, trifluoroacetic acid or triflic acid. The anhydrides can be either symmetrical (formed by the condensation of two molecules of the same carboxylic acid) or unsymmetrical (formed by the condensation of two different carboxylic acids).

As used herein, numerical ranges are meant to be inclusive of their upper and lower limits.

### Description of the Invention

The present invention provides several new processes for the nitration of aromatic substrates. The processes can be used for the nitration of a wide variety of aromatic substrates to selectively produce either mononitrated products or polynitrated products depending upon the process, the selection of particular reagents and reaction conditions as described more fully below. Further advantages of these processes include the mild nature of the process when compared with the usual mixed acid nitration processes, advantages in terms of non-corrosive conditions, low energy requirements, minimized oxidatin side products and generally better yields. In many cases the transformations are almost quantitative. Additionally, where isomers can be formed, the more valuable isomer from the industrial standpoint will frequently predominate.

For example, nitration of toluene using the process of the present invention produces a mixture of at least 9:1 of 2,4- and 2,6-dinitrotoluene, against 8:2 when using mixed acid. See, Josef Meissner GmbH & Co., DE 2 921 487 (1979). In the case of naphthalene, the yields of 1,5- and 1,8-dinitronaphthalene are similar to those obtained industrially either by direct mixed acid nitration of naphthalene, or by nitration of 1-nitronaphthalene. However, the process of the present invention has an advantage that much lower temperatures are used (compare, H. Dressier *in Kirk-Othmer Encyclopedia of Chemical Technology,* 3^{rd} Ed., Wiley Interscience, NY, 1981, Vol. 15, p. 724 and H. A. Lubs (Ed.), *The Chemistry of Synthetic Dyes and Pigments,* ACS Monograph No. 127, Reinhold Publ. Co., NY, 1955, p. 71-73).

Moreover, in the mixed acid processes of nitration, the nitrated products can undergo violent oxidative degradation, since nitric acid is a powerful oxidizing agent, especially at high temperatures. This disadvantage is minimized in the process of the present invention since it need not be carried out above room temperature.

Another advantage to the present invention is the ability to provide a direct nitration of such aromatic compounds as aniline and phenol. Industrially, nitrophenols are not obtained directly from phenol but must be synthesized indirectly. Thus, 2-chloronitrobenzene must first be prepared, then converted to the corresponding 2-nitrophenol. Similarly, 3-nitrophenol and 4-nitrophenol are prepared from 3-nitroaniline and 4-chloronitrobenzene, respectively, while 2,4-dinitrophenol is produced by hydrolysis of 2,4-dinitrochlorobenzene at 95-100°C. Additionally, the industrial mononitration of cresols is also indirect (*e.g.,* 2-nitro-p-cresol is produced from p-toluidine by diazotization followed by hydrolysis). See *Ullmann's Encyclopedia of Industrial Chemistry,* 5^{th} Ed., VCH, NY, 1987, Vol. A17, p. 446-451.

A further advantage to the present invention is the ability to provide a direct nitration of heteroaromatic compounds, in some instances without the need for any added nitric acid. For example, nitration of furfural according to one process of the present invention gives directly a high yield of 5-nitro-2-furaldehyde diacetate. Industrially, furfural is nitrated with nitric acid in acetic anhydride solution at 25-40°C to give 5-nitro-2-furaldehyde diacetate, used directly for the production of derived drugs. See Sanders, *et al., Ind. Eng. Chem.,* **47**:358-367 (1955) and *Ullmann's Encyclopedia of Industrial Chemistry,* 5^{th} Ed., VCH, NY, 1987, Vol. A17, p. 452.

In its most general application, the present invention provides a process for the nitration of aromatic compounds using the steps:
i) preparing an activated mixture of a silicate, an acid anhydride and an organic solvent;
ii) combining the aromatic substrate with the activated mixture to produce a reaction mixture;
iii) adding nitric acid to the reaction mixture; and
iv) isolating the nitrated aromatic compound from the mixture of step iii).

In the present inventive process, the first step is the formation of an activated mixture of a silicate, an acid anhydride and an organic solvent. The silicate used provides an inorganic solid acidic support for reagents which carry out the nitration of the aromatic substrates. These supports typically have extensive specific areas upon which the reagents and substrates are deposited. The precise nature of the silicate is not critical, however silicates such as clays, phyllosilicates and aluminosilicates are preferred. A particularly preferred silicate is montmorillonite clay. In certain preferred embodiments, the silicate is modified with a metal nitrate. The type of metal nitrate and the amount can vary greatly depending upon a particular application. Preferred metal nitrates are aluminum nitrate, bismuth nitrate, cadmium nitrate, cerium nitrate, cobalt nitrate, chromium nitrate, manganese nitrate, iron nitrate, nickel nitrate, zinc nitrate or copper nitrate. More preferred is a silicate modified with copper nitrate. A particularly preferred silicate is montmorillonite clay which is modified with copper nitrate (*claycop*). The amounts of metal nitrate with which a silicate is modified can be from 30 to 150% by weight, preferably 100% by weight.

The acid anhydrides are typically those which are commercially available and inexpensive. Preferred acid anhydrides are acetic anhydride, propionic anhydride, trifluoroacetic anhydride and triflic anhydride. Particularly preferred is acetic anhydride.

Organic solvents used in the present inventive process can be a hydrocarbon solvent such as hexane or cyclohexane, an ether, or a chlorinated hydrocarbon solvent. Preferred solvents are chlorinated hydrocarbon solvents such as chloroform, carbon tetrachloride, methylene chloride, trichloroethylene, tetrachloroethylene, 1,1-dichloroethane and 1,2-dichloropropane, with carbon tetrachloride being particularly preferred.

The activated mixture of step i) can be prepared by combining the organic solvent, the acid anhydride and the silicate. The precise order of addition can be varied. In a preferred method, the solvent is first added to the silicate at room temperature followed by addition of the acid anhydride. The resulting mixture is optionally stirred for a period of 15 minutes to 45 minutes.

In the second step of the present process, the activated mixture is combined with the aromatic substrate to provide a reaction mixture. The order of addition is not critical, however the addition of the substrate to the activated mixture provides the advantage of "one-pot" preparation. The resulting reaction mixture will typically be stirred for periods of from fifteen minutes to six hours, depending on the degree of nitration desired and the nature of the aromatic substrate.

In the third step of the process, nitric acid is added to the reaction mixture. Prior to the addition of nitric acid, the reaction mixture will optionally be cooled to 0°C to 5°C. The addition of nitric acid facilitates the nitration process by increasing both the rate of reaction and the yield of nitrated product. The amounts of nitric acid used will often be determined empirically depending upon the reactivity of the aromatic substrate and whether mono- or polynitration is desired. Dinitration of strongly activated aromatic substrates, such as cresols, will typically require from 0.05 mL to 1.0 mL of nitric acid per millimole of substrate, with from 0.20 mL to 0.40 mL per millimole of substrate being preferred. Dinitration of activated aromatic substrates, such as toluene, will typically use slightly larger amounts of nitric acid, preferably 0.50 mL to 1.5 mL per millimole of substrate. Similar amounts are preferred for the mononitration of deactivated aromatic substrates, such as chlorobenzene. Following the addition of nitric acid, the mixture will typically be stirred for a period of time of from 1 to 6 hours, preferably at room temperature. The precise lenght of time will be determined empirically and will depend upon the substrate and the temperature. Particular methods for determining the precise length of time are well know to those of skill in the art and include monitoring the progress of a reaction by gas chromatography, high performance liquid chromatography or thin layer chromatography, or by coupled techniques such as gas chromatography/mass spectrometry (GC/MS).

The process concludes with isolating the nitrated aromatic compound from the reaction mixture. The method of isolation is not critical and can be multistep. In a preferred embodiment, the reaction mixture is filtered and the filtrate cake is washed with solvent. The combined solvent and filter cake washes can be washed with water and dried using any conventional drying agent which is compatible with the particular solvent. Preferred drying agents are sodium sulfate, magnesium sulfate or molecular sieves. After removing the drying agent from the solution, the solvent is removed by evaporation under reduced pressure to provide the isolated crude nitrated aromatic compound. One of skill in the art can readily appreciate that in some instances the nitrated aromatic compound will crystallize in the filtrate and can be separated in a subsequent filtration. Alternatively, the solution can be concentrated and subjected to chromatography to provide the nitrated aromatic compound.

In a preferred embodiment, the present invention provides a process for the nitration of an activated aromatic substrate to produce a dinitrated aromatic compound. This process uses steps i) through iv) as described above, wherein the acid anhydride is acetic anhydride and the silicate is modified with copper nitrate.

In another preferred embodiment, the present invention provides a process for the nitration of an activated aromatic substrate to produce a mononitrated aromatic compound. This process uses steps i) through iv) as described above, wherein the acid anhydride is acetic anhydride and the silicate is modified with copper nitrate.

In yet another preferred embodiment, the present invention provides a process for the nitration of an unactivated aromatic substrate to produce a dinitrated aromatic compound. This process uses steps i) through iv) as described above, wherein the acid anhydride is acetic anhydride and the silicate is modified with copper nitrate.

In still another preferred embodiment, the present invention provides a process for the nitration of an unactivated aromatic substrate to produce a mononitrated aromatic compound. This process uses steps i) through iv) as described above, wherein the acid anhydride is acetic anhydride and the silicate is modified with copper nitrate.

In another preferred embodiment, the present invention provides a process for the nitration of a deactivated aromatic substrate to produce a mononitrated aromatic compound. This process uses steps i) through iv) as described above, wherein the acid anhydride is acetic anhydride and the silicate is modified with copper nitrate.

One of skill in the art can appreciate that the present inventive process is equally useful in the nitration of aromatic substrates having a combination of activating and deactivating substituents. For example, the dinitration of p-cresol proceeds through an intermediate mononitro-p-cresol which has both activating (-OH, -CH₃) and deactivating (-NO₂) substituents.

In another aspect, the present invention provides a process for the nitration of a strongly activated aromatic substrate using the steps:
i) preparing an activated mixture of an acid anhydride, an organic solvent and a silicate modified with copper nitrate;
ii) combining the strongly activated aromatic substrate with the activated mixture to produce a reaction mixture for a period of time sufficient to form a nitrated aromatic compound; and
iii) isolating the nitrated aromatic compound from the reaction mixture.

Preferred silicates, acid anhydrides and solvents, as well as amounts of copper nitrate are as described for the above processes. Additionally, one of skill in the art will recognize that considerations such as the order of addition of reagents, temperature ranges, and methods of isolating the nitrated products are all variable as described above.

However, in this process the degree of nitration (mononitration or polynitration) will depend upon the amount of modified clay which is used as well as the length of time the substrate is in contact with the activated mixture. To produce a mononitrated aromatic compound, the preferred amount of modified silicate is 100 to 300 milligrams per millimole of stronlgy activated aromatic substrate. To produce a dinitrated aromatic compound, the preferred amount of modified silicate is 200 to 500 milligrams per millimole of strongly activated aromatic substrate. The period of time which is required to effect the mononitration of a strongly activated aromatic substrate ranges from 30 min to 2 hr, while the length of time to achieve dinitration is typically from 1 to 6 hr. Particular methods for determining the precise length of time are well know to those of skill in the art and include monitoring the progress of a reaction by gas chromatography, high performance liquid chromatography, thin layer chromatography, or GC/MS.

The present invention further provides a process for the polynitration of a strongly aromatic substrate by the following steps:
i) preparing an activated mixture of a silicate modified with a metal nitrate, an acid anhydride and an organic solvent;
ii) combining the strongly activated aromatic substrate with the activated mixture to produce a reaction mixture for a period of time sufficient to form a polynitrated aromatic compound; and
iii) isolating the polynitrated aromatic compound from the reaction mixture.

In preferred embodiments, the silicate, metal nitrate, acid anhydride, organic solvent, and method of isolating the polynitrated aromatic compounds are as described in foregoing processes. The period of time which is required to effect the polynitration of a strongly activated aromatic substrate will be determined empirically and will depend upon the substrate and the temperature. At higher temperatures, shorter periods of time will be required. For strongly activated aromatic compounds such as p-cresol, a reaction time of 0.5 to 1.5 hours at room temperature will provide dinitro-p-cresol. Particular methods for determining the precise period of time are well known to those of skill in the art and include monitoring the progress of a reaction by gas chromatography, high performance liquid chromatography, thin layer chromatography or GC/MS.

The process of the present invention is illustrated in the following Examples for the production of several mono- and dinitroaromatic compounds as well as the trinitroaromatic musk xylol.

### EXAMPLES

### Reagents

Reagents and aromatic substrates used in the examples below are available from commercial sources. Silicates such as montmorillonite clay (K 10) are available from Aldrich Chemical Company (Milwaukee, Wisconsin, USA) or from Sud-Chemie AG (Munich, Germany). Silicates modified with metal nitrates can be prepared by literature methods. See, Cornélis and Laszlo, *Synthesis,* 909-918 (1985); Laszlo and Vandormael, *Chem. Lett.,* 1843-1846 (1988); and Cornélis, *et al., Tetrahedron Lett.,* **29**:5657-5660 (1988). Briefly, preparations for clays modified with copper nitrate (*claycop*) and clays modified with iron nitrate (*clayfen*) are as follows:

### Claycop

Montmorillonite clay (K 10, 30 g) is added to a solution of copper(II) nitrate trihydrate (28 g) in 375 mL of acetone. The resulting suspension is placed on a rotary evaporator and the solvent is removed under reduced pressure with gentle heating (water bath at 50°C). After 30 min, the dry solid crust adhering to the walls of the flask is flaked off and powdered. Evaporation is continued for another 30 min to produce claycop as a light blue, free-flowing powder (58 g). Claycop may be prepared and stored for up to three months prior to use.

### Clayfen

In a manner similar to the procedure used for claycop, iron(III) nitrate nonahydrate (22.5 g) is dissolved in 375 mL of acetone to form a homogeneous rusty brown solution which then turns into a muddy, light brown suspension. To this suspension is added K 10 clay (30 g) in small amounts with stirring. Solvent is removed as described above to yield K 10 clay-supported iron(III) nitrate *(clayfen)* as a yellow, floury powder *(ca.* 50 g).

Clayfen is best prepared immediately prior to use and should not be stored in a closed flask.

### Conditions

Unless otherwise noted all reactions were carried out at room temperature on a 10 mmol scale. Isomers were separated by crystallization, typically from an alcohol such as methanol or ethanol, or by column chromatography on silica gel using mixtures of n-hexane and ethyl acetate as eluant

### EXAMPLE 1

This example illustrates the preparation of 1,3-dinitrobenzene as an example of the dinitration of an unactivated aromatic substrate.

Benzene (0.78 g, 10 mmol) was added to a suspension of claycop (4.8 g) in carbon tetrachloride (30 mL) and acetic anhydride (15 mL). After 1.5-2 h, the reaction mixture was cooled in an ice bath and nitric acid (10 mL) was added in small portions. After 1-2 h, the reaction mixture was cooled again and more acid (10 mL) added in small portions. After addition of the nitric acid, the reaction mixture was kept at room temperature for 8 h, then filtered. The filter cake was washed with solvent and the combined organic portions were washed with water and dried over anhydrous sodium sulphate, filtered and concentrated under reduced pressure to provide 1,3-dinitrobenzene in 70% yield, which was identical with a sample from commercial sources.

### EXAMPLE 2

This example illustrates the preparation of 2,4-dinitrotoluene as an example of the dinitration of an activated aromatic substrate.

Toluene (0.92 g, 10 mmol) was added to a suspension of claycop (4.8 g) in carbon tetrachloride (30 mL) and acetic anhydride (15 mL). After 1.5-2 h, the reaction mixture was cooled in an ice bath and nitric acid (10 mL) was added in small portions. After addition of nitric acid, the reaction mixture was kept at room temperature for 4 h, then filtered. The filter cake was washed with solvent and the combined organic portions were washed with water and dried over anhydrous sodium sulphate, filtered and concentrated under reduced pressure to provide a mixture of crude dinitrotoluenes in 85% overall yield, from which were isolated 2,6-dinitrotoluene (10% of the product mixture) and 2,4-dinitrotoluene (90% of the product mixture), which were identical with commercial samples.

### EXAMPLE 3

This example illustrates the preparation of 1,8-dinitronaphthalene as an example of the dinitration of an unactivated two fused-ring aromatic substrate.

The procedure of Example 2 was followed, using naphthalene. After 3.5 h, the reaction mixture was filtered and the filter cake was washed with solvent. The organic portions were combined, washed with water, dried over anhydrous sodium sulphate, filtered and concentrated under reduced pressure to provide 1,8-dinitronaphthalene (65% yield) and 1,5-dinitronaphthalene (30% yield). Only traces of 1,6-dinitronaphthalene and 1,7-dinitronaphthalene were detected by GC/MS analysis. Physical data for the isolated compounds was identical with literature data and those samples from commercial sources.

### EXAMPLE 4

This example illustrates the preparation of 12,14-dinitrodehydroabietic acid and methyl 12,14-dinitrodehydroabietate as an example of the dinitration of an activated hindered polycyclic aromatic diterpenoid.

The procedure of Example 2 was followed, using dehydroabietic acid, isolated from commercial disproportionated rosin or produced by disproportionation of commercial rosin, and methyl dehydroabietate, obtained by methylation of dehydroabietic acid with diazomethane. After 2.5 h, the reaction mixtures were treated as described in Example 2 to provide 12,14-dinitrodehydroabietic acid (85% yield) and methyl 12,14-dinitrodehydroabietate (87% yield), respectively. These products were identical with samples prepared by conventional nitration using mixed acid.

### EXAMPLE 5

This example illustrates the preparation of musk tibetene as an example of the dinitration of an activated aromatic substrate.

The procedure of Example 2 was followed, using 5-t-butyl-1,2,3--trimethylbenzene. After the addition of nitric acid, the reaction mixture was stirred at room temperature for 2.5 h, then treated as described in Example 2 to provide musk tibetene, i.e. 5-t-butyl-4,6-dinitro-1,2,3-trimethylbenzene (5-t-butyl-dinitro-hemimellitene) as crystals (m.p. 135-136°C, 93% yield). Physical data for the isolated compound were identical with literature data.

### EXAMPLE 6

This example illustrates the preparation of musk xylol as an example of the trinitration of an activated aromatic substrate.

5-t-Butil-1,3-dimethylbenzene (1.62 g, 10 mmol) was added to a suspension of claycop (3.0 g) in carbon tetrachloride (30 mL) and acetic anhydride (15 mL). After 1.5-2 h, the reaction mixture was cooled in an ice bath and nitric acid (10 mL) was added in small portions. After 24 h and addition of nitric acid totaling 35 mL, the reaction mixture was filtered. The filter cake was washed with solvent. and the combined organic portions were washed with water, dried over anhydrous sodium sulfate, filtered and concentrated under reduced pressure to provide musk xylol, *i.e.* 5-t -butyl-1,3-dimethyltrinitrobenzene as crystals (m.p. 108-112°C, from ethanol in 82% yield). Physical data for the isolated compound were identical with literature data.

### EXAMPLE 7

This example illustrates the preparation of mononitrochlorobenzene as an example of the mononitration of a deactivated aromatic substrate.

Freshly prepared claycop (4.8 g) was suspended in a mixture of carbon tetrachloride (30 mL) and acetic anhydride (15 mL) and stirred at room temperature for 45 min. Chlorobenzene (1.12 g, 10 mmol) was added and stirring was continued for 2 hr. The resulting mixture was cooled to 0-5°C and nitric acid (15 mL) was slowly added. After 5-6 hr, the mixture was treated as described in Example 2 to provide a quantitative yield of nitrochlorobenzene as a mixture of isomers (*para:ortho:metha,* 85:13:2, as determined by GC/MS). Physical data for the isolated compounds were identical with literature data.

### EXAMPLE 8

This example illustrates the preparation of mononitrobenzaldehyde as an example of the mononitration of a deactivated aromatic substrate, wherein increased amounts of the para-nitro isomer are obtained.

Freshly prepared claycop (4.8 g) was suspended in a mixture of carbon tetrachloride (30 mL) and acetic anhydride (15 mL) and stirred at room temperature for 45 min. Benzaldehyde (1.06 g, 10 mmol) was added and stirring was continued for 2 hr. The resulting mixture was cooled to 0-5°C and nitric acid (10 mL) was slowly added. After 3 hr, the mixture was treated as described in Example 2 to provide a mixture of nitrobenzaldehydes *(para:ortho:metha,* 27%:26%:41%, as determined by GC/MS) in approximately 90% overall yield.

### EXAMPLE 9

This example illustrates the mono- and the dinitration of a strongly activated aromatic substrate.

Freshly prepared claycop (4.8 g) was suspended in a mixture of carbon tetrachloride (30 mL) and acetic anhydride (15 mL) and stirred at room temperature for 45 min. Aniline (0.93 g, 10 mmol) was added and stirring was continued. After 5 min, 2-nitroacetanilide had formed (as determined by GC/MS) as a result of acylation of the amino group. The reaction mixture was treated as described in Example 2 to provide 2-nitroacetanilide as yellow crystals (m.p. 98-100°C, from methanol in 90% yield) which was identical with a sample from commercial sources.

Addition of nitric acid (2 mL) to the reaction mixture, and treatment of the reaction mixture after 6 h as described in Example 2, provided crude dinitroacetanilide in 85% yield, which was crystallized from methanol to provide 2,4-dinitroacetanilide (yellow crystals, m.p. 120-122°C, 76% yield). Physical data for the isolated compound were identical with literature data.

### EXAMPLE 10

This example illustrates preparation of 2-nitro-p-cresol as an example of the mononitration of a strongly activated aromatic substrate using reduced amounts of a silicate modified with copper nitrate and in the absence of any added nitric acid.

Freshly prepared claycop (2.4 g) was suspended in a mixture of carbon tetrachloride (30 mL) and acetic anhydride (15 mL) and stirred at room temperature for 45 min. p-Cresol (1.08 g, 10 mmol) was added and stirring was continued at room temperature for 15 min. The resulting mixture was filtered, washed, dried and concentrated in the usual manner to provide 2-nitro-p-cresol in 98% yield, which was isolated by crystallization (m.p. 33-35°C, methanol/ether, 80% yield), identical with a sample from commercial sources.

### EXAMPLE 11

This example illustrates the preparation of 2,6-dinitro-p-cresol as an example of the dinitration of a strongly activated aromatic substrate using a silicate modified with copper nitrate and in the absence of any added nitric acid.

An activated mixture of freshly prepared claycop, carbon tetrachloride and acetic anhydride was prepared as described in Example 7. To the activated mixture was added p-cresol (1.08 g, 10 mmol) and stirring was continued at room temperature for 4 hr. The resulting mixture was filtered, washed, dried and concentrated in the usual manner to provide a 79% yield of 2,6-dinitro-p-cresol (m.p. 77-79°C, from methanol).

Similar treatment of o-cresol provided 4,6-dinitro-o-cresol in 58% yield.

Physical data for the isolated compounds were identical with those of samples from commercial sources.

### EXAMPLE 12

This example illustrates preparation 2,6-dinitro-p-cresol as an example of the dinitration of a strongly activated aromatic substrate.

Freshly prepared claycop (4.8 g) was suspended in a mixture of carbon tetrachloride (30 mL) and acetic anhydride (15 mL) and stirred at room temperature for 45 min. p-Cresol (1.08 g, 10 mmol) was added and stirring was continued at room temperature for 15 min. Addition of nitric acid (0.5 mL) to the reaction mixture, and treatment of the reaction mixture after 0.5 hr as described in Example 2, provided 2,6-dinitro-p-cresol in 95% yield, which was isolated by crystallization (m.p. 78°C, methanol/ether, 80% yield), identical with a sample from commercial sources.

For o-cresol, 2.5 mL of nitric acid were required to produce 4,6-dinitro-o-cresol in 95% yield.

### EXAMPLE 13

This example illustrates preparation of mononitroanisoles an example of the mononitration of a strongly activated aromatic substrate using reduced amounts of a silicate modified with copper nitrate and in the absence of any added nitric acid.

Freshly prepared claycop (2.4 g) was suspended in a mixture of carbon tetrachloride (30 mL) and acetic anhydride (15 mL) and stirred at room temperature for 45 min. Anisole (1.08 g, 10 mmol) was added and stirring was continued at room temperature for 15 min. The resulting mixture was filtered, washed, dried and concentrated in the usual manner to provide a mixture of 2-nitro-anisole in 44% yield, and 4-nitro-anisole in 52% yield (as determined by GC/MS), identical to samples from commercial sources.

### EXAMPLE 14

This example illustrates the mononitration of furfural as an example of the mononitration of a five-membered aromatic heterocycle.

An activated mixture of freshly prepared claycop, carbon tetrachloride and acetic anhydride was prepared as described in Example 7. To the activated mixture was added furfural (0.96 g, 10 mmol) and stirring was continued at room temperature for 2 hr. The resulting mixture was filtered, washed, dried and concentrated in the usual manner to provide a 65% yield of mononitrofurfurylidene diacetate (as determined by GC/MS).

Addition of nitric acid (3 mL) to the reaction mixture as in Example 2 did not change the composition of the reaction product nor the overall yield.

### EXAMPLE 15

This example illustrates the mononitration and the dinitration of thiophenecarboxyaldehyde.

An activated mixture of freshly prepared claycop, carbon tetrachloride and acetic anhydride was prepared as described in Example 7. To the activated mixture was added thiophenecarboxyaldehyde (1.12 g, 10 mmol) and stirring was continued at room temperature for 2hr. The reaction mixture was filtered, dried and concentrated in the usual manner to provide a 75% yield of mononitrothiophenecarboxyaldehyde diacetate (as determined by MS).

Addition of nitric acid (2 mL) to the reaction mixture as in Example 2 provided a quantitative yield of dinitrothiophenecarboxyaldehyde diacetate (as determined by MS).

### EXAMPLE 16

This example illustrates the mononitration and the dinitration of thiophene.

An activated mixture of freshly prepared claycop, carbon tetrachloride and acetic anhydride was prepared as described in Example 7. To the activated mixture was added thiophene (0.84 g, 10 mmol) and stirring was continued at room temperature for 30 min. The reaction mixture was filtered, washed, dried and concentrated in the usual manner to provide a 98% yield of mononitrothiophene (as determined by MS).

Addition of nitric acid (5 mL) as in Example 2 provided a 90% yield of dinitrothiophene as 6:4 mixture of isomers (as determined by GC/MS).

## Claims

1. A process for the nitration of an aromatic substrate, wherein activated and unactivated aromatic substrates are polynitrated and deactivated aromatic substrates are either mononitrated or polynitrated, comprising the steps;
i) preparing an activated mixture of a silicate, an acid anhydride and an organic solvent;
ii) combining said aromatic substrate with said activated mixture to produce a reaction mixture;
iii) adding nitric acid to said reaction mixture; and
iv) isolating said nitrated aromatic compound from said mixture of step iii).

2. A process in accordance with claim 1 wherein said silicate is modified with a metal nitrate selected from the group consisting of aluminum nitrate, bismuth nitrate, cadmium nitrate, cerium nitrate, cobalt nitrate, chromium nitrate, manganese nitrate, iron nitrate, nickel nitrate, zinc nitrate and copper nitrate.

3. A process in accordance with claim 1 wherein said silicate is modified with copper nitrate.

4. A process in accordance with claim 1 wherein said silicate is selected from the group consisting of clay, phyllosilicate, and aluminosilicate.

5. A process in accordance with claim 1 wherein said silicate is montmorillonite clay modified with copper nitrate.

6. A process in accordance with claim 1 wherein said organic solvent is selected from the group consisting of chloroform, carbon tetrachloride, methylene chloride, trichloroethylene, tetrachloroethylene, 1,1-dichloroethane and 1,2-dichloropropane.

7. A process in accordance with claim 1 wherein said acid anhydride is selected from the group consisting of acetic anhydride, propionic anhydride, trifluoroacetic anhydride and triflic anhydride.

8. A process in accordance with claim 1 wherein said aromatic substrate is an activated aromatic compound, said nitrated aromatic compound is a dinitrated aromatic compound, said acid anhydride is acetic anhydride and said silicate is modified with copper nitrate.

9. A process in accordance with claim 1 wherein said aromatic substrate is an unactivated aromatic compound, said nitrated aromatic compound is a dinitrated aromatic compound, said acid anhydride is acetic anhydride and said silicate is modified with copper nitrate.

10. A process in accordance with claim 1 wherein said aromatic substrate is a deactivated aromatic compound, said nitrated aromatic compound is a mononitrated aromatic compound, said acid anhydride is acetic anhydride and said silicate is modified with copper nitrate.

11. A process in accordance with claim 1 wherein said aromatic substrate has both activating and deactivating substituents, said acid anhydride is acetic anhydride and said silicate is modified with copper nitrate.

12. A process for the nitration of a strongly activated aromatic substrate comprising the steps;
i) preparing an activated mixture of an acid anhydride, an organic solvent and a silicate modified with copper nitrate;
ii) combining said strongly activated aromatic substrate with said activated mixture to produce a reaction mixture for a period of time sufficient to form a nitrated aromatic compound; and
iii) isolating said nitrated aromatic compound from said reaction mixture.

13. A process in accordance with claim 12 wherein said silicate modified with copper nitrate is present in an amount of 100 to 300 milligrams per millimole of said aromatic substrate and said nitrated aromatic compound is a mononitrated aromatic compound.

14. A process in accordance with claim 12 wherein said silicate modified with copper nitrate is present in an amount of 200 to 500 milligrams per millimole of said aromatic substrate and said nitrated aromatic compound is a dinitrated aromatic compound.

15. A process for the polynitration of a strongly activated aromatic substrate comprising the steps;
i) preparing an activated mixture of an acid anhydride, an organic solvent and a silicate modified with a metal nitrate;
ii) combining said strongly activated aromatic substrate with said activated mixture to produce a reaction mixture for a period of time sufficient to form a polynitrated aromatic compound; and
iii) isolating said polynitrated aromatic compound from said reaction mixture.

16. A process in accordance with claim 15 wherein said silicate modified with a metal nitrate is montmorillonite clay modified with copper nitrate.

## Patentansprüche

1. Verfahren zur Nitrierung von einem aromatischen Substrat, worin die aktivierte und unaktivierte aromatische Substrate polynitriert sind und die deaktivierte aromatische Substrate mononitriert oder polynitriert sind, gekennzeichnet durch die Stufe:
i) Herstellung einer aktivierte Mischung bestehend aus einer Silikat, einer Säureanhydrid und einen organischen Lösungsmittel;
ii) Kombination vorgenannt aromatische Substrat mit vorgenannt aktivierte Mischung zur Herstellung eines Reaktionsgemisch;
iii) Zusatz von Salpetersäure zu vorgenannten Reaktionsgemisch; und
iv) Abtrennung vorgenannte nitrierte aromatische Verbindung aus vorgenannte Mischung aus Stufe iii).

2. Verfahren nach Anspruch 1, worin vorgenannte Silikat mit einem metallischen Nitrat, aus der Gruppe Aluminiumnitrat, Bismuthnitrat, Cadmiumnitrat, Ceriumnitrat, Cobaltnitrat, Chromnitrat, Mangannitrat, Eisennitrat, Nickelnitrat, Zinknitrat und Kupfernitrat überführt wird.

3. Verfahren nach Anspruch 1, worin vorgenannte Silikat mit Kupfernitrat überführt ist.

4. Verfahren nach Anspruch 1, worin vorgenannt Silikat aus der Gruppe Ton, Phyllosilikat und Aluminosilikat ausgewählt wird.

5. Verfahren nach Anspruch 1, worin vorgenannt Silikat Montmorillonit Ton ist, überführt mit Kupfernitrat.

6. Verfahren nach Anspruch 1, worin vorgenannt organischen Lösungsmittel aus der Gruppe Chloroform, Carbontetrachlorid, Methylenechlorid, Trichlorethylen, Tetrachlorethylen, 1,1-Dichlorethan und 1,2-Dichlorpropan ausgewählt wird.

7. Verfahren nach Anspruch 1, worin vorgenannt Säureanhydrid aus der Gruppe Essigsäureanhydrid, Propansäureanhydrid, Trifluoressigsäureanhydrid, und Trifluormethansulfonsäureanhydrid ausgewählt wird.

8. Verfahren nach Anspruch 1, worin das vorgenannte aromatische Substrat eine aktivierte aromatische Verbindung ist, vorgenannte nitrierte aromatische Verbindung eine dinitrierte aromatische Verbindung ist, vorgenanntes Säureanhydrid Essigsäureanhydrid ist und vorgenanntes Silikat mit Kupfernitrat überführt wird.

9. Verfahren nach Anspruch 1, worin das vorgenannte aromatische Substrat eine unaktivierte aromatische Verbindung ist, vorgenannte nitrierte aromatische Verbindung eine dinitrierte aromatische Verbindung ist, vorgenanntes Säureanhydrid Essigsäureanhydrid ist und vorgenanntes Silikat mit Kupfernitrat überführt wird.

10. Verfahren nach Anspruch 1, worin das vorgenannte aromatische Substrat eine deaktivierte aromatische Verbindung ist, vorgenannte nitrierte aromatische Verbindung eine mononitrierte aromatische Verbindung ist, vorgenanntes Säureanhydrid Essigsäureanhydrid ist und vorgenanntes Silikat mit Kupfernitrat überführt wird.

11. Verfahren nach Anspruch 1, worin das vorgenannte aromatische Substrat die beide aktivierende und deaktivierende Substituente hat, das Säureanhydrid Essigsäureanhydrid ist und vorgenanntes Silikat mit Kupfernitrat überführt wird.

12. Verfahren zur Nitrierung eines starke aktivierte aromatische Substrat, gekennzeichnet durch die Stufe:
i) Vorbereitung einer aktivierten Mischung aus einem Säureanhydrid, einem organischen Lösungsmittel und einem mit Kupfernitrat übergeführten Silikat;
ii) Kombination vorgenannt stark aktivierte aromatische Substrat mit vorgenannt aktivierte Mischung zur Herstellung eines Reaktionsgemisch für einen genügenden Zeitraum, um eine nitrierte aromatische Verbindung zur herstellen; und
iii) Abtrennung vorgenannte nitrierte aromatische Verbindung aus vorgenannte Reaktionsgemisch

13. Verfahren nach Anspruch 12, worin vorgenannte mit Kupfernitrat übergeführte Silikat 100-300 Milligram per Millimole von vorgenannten aromatischen Substrat beträgt und vorgenannte nitrierte aromatische Verbindung eine mononitrierte aromatische Verbindung ist.

14. Verfahren nach Anspruch 12, worin vorgenannte mit Kupfernitrat übergeführte Silikat 200-500 Milligram per Millimole von vorgenannten aromatischen Substrat beträgt und vorgenannte nitrierte aromatische Verbindung eine dinitrierte aromatische Verbindung ist.

15. Verfahren zur Polynitrierung eines starke aktivierte aromatische Substrat, gekennzeichnet durch die Stufe:
i) Vorbereitung einer aktivierten Mischung aus einem Säureanhydrid, einem organischen Lösungsmittel und einem mit einem metallischen Nitrat übergeführten Silikat;
ii) Kombination vorgenannt starke aktivierte aromatische Substrat mit vorgenannte aktivierte Mischung zur Herstellung eines Reaktionsgemisch für einen genügenden Zeitraum, um eine polynitrierte aromatische Verbindung zur herstellen; und
iii) Abtrennung vorgenannte polynitrierte aromatische Verbindung aus vorgenannte Reaktionsgemisch

16. Verfahren nach Anspruch 15, worin das vorgenannte mit einem metallischen Nitrat übergeführte Silikat mit Kupfernitrat übergeführte Montmorillonite Ton ist.

## Revendications

1. Un procédé pour la nitration d'un substrat aromatique, où des substrats aromatiques activés et unactivés sont polynitrés et des substrats aromatiques deactivés sont mononitrés ou polynitrés, comprenant les étapes de;
i) préparation d'un mélange activé d'un silicate, un anhydride d'acide et un solvant organique;
ii) combinaison du substrat avec le mélange activé pour produire le mélange de réaction;
iii) addition d'acide nitrique au mélange de réaction; et
iv) isolement du composé nitré du mélange de réaction de l'étape iii).

2. Un procédé selon la revendication 1 où le silicate est modifié avec un nitrate de métal sélectionné du groupe qui consiste en nitrate d'aluminium, nitrate de bismuth, nitrate de cadmium, nitrate de cérium, nitrate de cobalt, nitrate de chrome, nitrate de manganèse, nitrate de fer, nitrate de zinc et nitrate de cuivre.

3. Un procédé selon la revendication 1 où le silicate est modifié avec du nitrate de cuivre.

4. Un procédé selon la revendication 1 où le silicate est sélectionné du groupe qui consiste en argile, phyllosilicate, et aluminosilicate.

5. Un procédé selon la revendication 1 où le silicate est de l'argile montmorillonite modifiée avec du nitrate de cuivre.

6. Un procédé selon la revendication 1 où le solvent organique est sélectionné du groupe qui consiste en chloroforme, tetrachlorure de carbone, chlorure de méthylène, trichloréthylène, tetrachloréthylène, 1,1-dichloréthane et 1,2-dichloropropane.

7. Un procédé selon la revendication 1 où l'anhydride d' acide est sélectionné du groupe qui consiste en anhydride acétique, anhydride propionique, anhydride trifluoroacétique et anhydride triflique.

8. Un procédé selon la revendication 1 où le substrat aromatique est um composé aromatique activé, le composé aromatique nitré est um composé aromatique dinitré, l' anhydride est de l'anhydride acétique et le silicate est modifié avec du nitrate de cuivre.

9. Un procédé selon la revendication 1 où le substrat aromatique est um composé aromatique unactivé, le composé aromatique nitré est um composé aromatique dinitré, l' anhydride est de l'anhydride acétique et le silicate est modifié avec du nitrate de cuivre.

10. Un procédé selon la revendication 1 où le substrat aromatique est um composé aromatique desactivé, le composé aromatique nitré est um composé aromatique mononitré, l' anhydride est de l'anhydride acétique et le silicate est modifié avec du nitrate de cuivre.

11. Un procédé selon la revendication 1 où le substrat aromatique possède à la fois des substituants activantes et déactivantes, l'anhydride est de l'anhydride acétique et le silicate est modifié avec du nitrate de cuivre.

12. Un procédé pour la nitration d'un substrat aromatique fortement activé comprenant les étapes de;
i) preparation d'un mélange activé d'un anhydride d' acide, un solvant organique et d'un silicate modifié avec du nitrate de cuivre;
ii) combinaison du substrat aromatique fortement activé avec le mélange activé pendant une période de temps suffisante pour former um composé aromatique nitré; et
iii) isolement du composé nitré du mélange de réaction.

13. Un procédé selon la revendication 12 où le silicate modifié avec du nitrate de cuivre est present dans une quantité comprise entre 100 et 300 milligrammes pour millimole de substrat aromatique et le composé aromatique nitré est um composé aromatique mononitré.

14. Un procédé selon la revendication 12 où le silicate modifié avec du nitrate de cuivre est present dans une quantité comprise entre 200 et 500 milligrammes pour millimole du substrat aromatique et le composé aromatique nitré est um composé aromatique dinitré.

15. Un procédé pour la polynitration d'un substrat aromatique fortement activé comprenant les étapes de;
i) préparation d'un mélange activé d'un anhydride d'acide, un solvant organique et d'un silicate modifié avec un nitrate de métal;
ii) combinaison du substrat aromatique fortement activé avec le mélange activé pour produire un mélange de réaction pendant une période de temps suffisante pour former um composé aromatique polynitré, et
iii) isolement du composé nitré du mélange de réaction.

16. Un procédé selon la revendication 15 où le silicate modifié avec un nitrate de métal est de l'argile montmorillonite modifiée avec du nitrate de cuivre.
